# EUROPEAN PATENT APPLICATION

(11) **EP 3 708 191 A1**
(43) Date of publication of application: **16.09.2020**
(21) Application number: 18873538.5
(22) Date of filing: 19.10.2018
(51) Int. Cl.: A61K 49/14, C12N 5/07, C12N 11/02

(54) **COMPOSITE PARTICLES FOR IMAGING, METHOD FOR PRODUCING COMPOSITE PARTICLES, CELLS, CELL STRUCTURE, AND MIXED DISPERSION**

(30) Priority: 06.11.2017 JP 2017213709
(71) Applicant: Konica Minolta, Inc., Tokyo 100-7015 (JP); Tabata, Yasuhiko, Uji-shi Kyoto 611-0024 (JP)
(72) Inventor: TABATA, Yasuhiko, Uji-city Kyoto 611-0024 (JP); INUI, Chie, Tokyo 100-7015 (JP); MAEZAWA, Akihiro, Tokyo 100-7012 (JP); MOCHIZUKI, Makoto, Tokyo 100-7015 (JP); HIRAYAMA, Natsumi, Tokyo 100-7015 (JP)
(74) Representative: Henkel & Partner mbB
(86) International application number: PCT/JP2018/039058
(87) International publication number: WO 2019/087828

(57) **Abstract**

The purpose of the present invention is to provide composite particles for imaging that have high biodegradability after imaging. To achieve the above purpose, the composite particles for imaging according to the present invention are configured such that the ratio (long-term residual amount/short-term residual amount) of a long-term residual amount, which is the average value of the contrast rate after six days in a cell and the contrast rate after 11 days in a cell of the same type, and a short-term residual amount, which is the contrast rate after two days in a cell of the same type, is less than 99%.

## Description

### Technical Field

the present invention relates to a contrast composite particle, a method for producing a composite particle, a cell, a cell structure, and a mixed dispersion.

### Background Art

When an image of the inside of the living body is taken for diagnostic imaging, a particle having a contrast effect (hereinafter, simply referred to as "contrast agent particle".) is used in order to more clearly express an observation object on the image. Examples of such a contrast agent particle include a particle of a magnetic material (iron oxide or the like) as a contrast agent for MRI. Such a contrast agent particle may be sometimes used as a particle including a biodegradable substance (hereinafter, simply referred to as "composite particle".) in order to achieve an object of enhancements in stability and biological compatibility in the living body.

For example, PTL 1 describes a composite particle obtained by annealing a mixed powder including a magnetic iron oxide nanoparticle surface-treated with a hydrophobic organic substance, and a salt particle, to thereby prepare an iron oxide nanoparticle including no organic substance, and thereafter coating the iron oxide nanoparticle with a hydrophilic polymer. PTL 1 describes the following: the mixed powder can be annealed to result in an enhancement in crystallinity of the iron oxide nanoparticle and an enhancement in magnetic properties of the iron oxide nanoparticle.

PTL 2 describes a composite particle obtained by mixing and stirring a magnetically responsive particles such as an iron oxide nanoparticles, and a protein such as gelatin. PTL 2 describes the following: the protein is high in biological compatibility and thus the composite particle is high in safety.

### Citation List

### Patent Literatures

PTL 1: Japanese Unexamined Patent Application Publication (Translation of PCT Application) No. 2014-511324
PTL 2: Japanese Unexamined Patent Application Publication (Translation of PCT Application) No. 2009-519894

### Summary of Invention

### Technical Problem

as described in PTL 1 and PTL 2, a composite particle has been conventionally studied where a contrast agent particle such as an iron oxide nanoparticle is coated with a hydrophilic polymer such as gelatin in order to enhance biological compatibility of a contrast agent before an image is taken. Such a composite particle is administered to a living body and allowed to arrive at cells present in an observation object, or is directly administered to cells as an observation object (hereinafter, such arriving of a composite particle to cells present in an observation object and such direct administration of a composite particle to cells as an observation object will be collectively referred to as "administer(in)".), and thereafter the hydrophilic polymer or the like is dissolved to thereby release the contrast agent particle.

Meanwhile, a material for use in a contrast agent particle, such as iron oxide, also encompasses a material low in biodegradability, and such a material may sometimes remain in a living body (cells) for a long period after an image is taken. A contrast agent particle after an image is taken is desirably early dissolved or degraded and excreted from the living body (cells). In other words, such a composite particle after an image is taken is desirably high in biodegradability.

An object of the present invention is to provide a contrast composite particle high in biodegradability after an image is taken, a method for producing the composite particle, a cell including the composite particle in the interior of a cell membrane, a cell structure including the cell, and a mixed dispersion including the cell or the cell structure.

### Solution to Problem

the object of the present invention is achieved by the following means.
[1] A contrast composite particle having a ratio between a long-term residual amount and a short-term residual amount (long-term residual amount/short-term residual amount) of less than 90%, wherein the long-term residual amount is an average of a contrast rate in cells after a lapse of 6 days and a contrast rate in cells of the same species after a lapse of 11 days and the short-term residual amount is a contrast rate in cells of the same species after a lapse of 2 days.
[2] The composite particle according to [1], comprising: a substance having a contrast effect; and a degradation accelerator that is a substance to be more easily dissolved or degraded in cells than the substance having a contrast effect and that is a substance to accelerate dissolution or degradation of the substance having a contrast effect; wherein the degradation accelerator is encapsulated in the substance having a contrast effect.
[3] The composite particle according to [2], wherein the substance having a contrast effect is a substance comprising an atom of a metal element, and the degradation accelerator comprises a compound comprising an atom of any metal element that is higher in ionization tendency than the metal element, or a chelating agent capable of being coordinately bound to the atom of the metal element.
[4] The composite particle according to [3], wherein a proportion of a mass of the atom of any metal element that is higher in ionization tendency than the metal element, based on a total mass of components other than water, is 0.1mass% or more and 90mass% or less.
[5] The composite particle according to [3] or [4], wherein the metal element is Fe, and any metal element that is higher in ionization tendency than the metal element is a compound comprising a metal element selected from the group consisting of Mg, Na, Ca, K, Al, and Zn.
[6] The composite particle according to [2], wherein the degradation accelerator comprises a biodegradable substance.
[7] The composite particle according to [6], wherein the biodegradable substance is gelatin.
[8] The composite particle according to [6] or [7], wherein a proportion of a mass of the biodegradable substance based on a total mass of components other than water is 5mass% or more and 95mass% or less.
[9] The composite particle according to [2], wherein the substance having a contrast effect is a substance comprising an atom of a metal element, and the degradation accelerator comprises a compound comprising an atom of any metal element that is higher in ionization tendency than the metal element, or a chelating agent capable of being coordinately bound to the atom of the metal element, and a biodegradable substance.
[10] The composite particle according to any one of [2] to [9], wherein the substance having a contrast effect is a substance capable of being comprised in a contrast agent for MRI.
[11] The composite particle according to [10], wherein the substance having a contrast effect comprises Fe, and a saturated magnetic amount per gram of Fe at a water content of 0.01mass% or more and 10mass% or less is 1 emu/g or more and 150 emu/g or less.
[12] The composite particle according to any one of [2] to [11], wherein a volume average particle size of the composite particle is 2.0 nm or more and 1500 nm or less.
[13] The composite particle according to any one of [2] to [12], wherein an outer edge of the composite particle is covered with the degradation accelerator.
[14] A contrast composite particle comprising: a substance having a contrast effect; and a degradation accelerator that is a substance to be more easily dissolved or degraded in cells than the substance having a contrast effect and that is a substance to accelerate dissolution or degradation of the substance having a contrast effect; wherein
   the degradation accelerator is encapsulated in the substance having a contrast effect.
[15] A contrast composite particle in which, in a case where a rate of decrease per unit time in concentration in living cells after administration to the living cell is measured with respect to two periods not overlapping with each other, the rate of decrease at a shorter elapsed time after administration is lower than the rate of decrease at a longer elapsed time after administration.
[16] A method for producing the composite particle according to any one of [1] to [15], comprising: preparing a solution comprising a substance having a contrast effect, and a degradation accelerator that is a substance to be more easily dissolved or degraded in cells than the substance having a contrast effect, and forming a particle of the substance having a contrast effect, in the solution.
[17] A cell comprising the composite particle according to any one of [1] to [15], in an interior of a cell membrane.
[18] A cell structure obtained by collecting a plurality of the cells according to [17].
[19] The cell structure according to[18], wherein the cell structure is a cell sheet where a plurality of the cells are aggregated in the form of a sheet, a spheroid where a plurality of the cells are aggregated in the form of a sphere, a structure where a plurality of the cells are covered with a film comprising a polymer, or a structure where a surface of a steric structure comprising a polymer is covered with a plurality of the cells.
[20] A mixed dispersion comprising a plurality of the cells according to [17], or the cell structure according to [18] or [19], and a polymer.

### Advantageous Effects of Invention

The present invention provides a contrast composite particle high in biodegradability after an image is taken, a method for producing the composite particle, a cell including the composite particle in the interior of a cell membrane, a cell structure including the cell, and a mixed dispersion including the cell or the cell structure.

### Brief Description of Drawings

[FIG. 1] FIG. 1A illustrates a schematic view of a composite particle according to one embodiment of the present invention, and FIG. IB illustrates a schematic view of a composite particle according to another embodiment of the present invention.
[FIG. 2] FIGS. 2A to 2D each illustrate a schematic view of a state of degradation of a composite particle according to one embodiment of the present invention, and FIG. 2A, FIG. 2B, FIG. 2C, and FIG. 2D illustrate respective schematic views of a composite particle administered into cells, a composite particle where a degradation accelerator is preferentially dissolved or degraded, a composite particle where dissolution or degradation of a contrast agent particle is also accelerated, and a composite particle to be acceleratedly dissolved or degraded.
[FIG. 3] FIG. 3 illustrates a schematic view of a composite particle according to another embodiment of the present invention.

### Description of Embodiments

The present inventors have made intensive studies in order to solve the above problems, and thus have produced a particle including a contrast agent and a biodegradable substance (hereinafter, simply referred to as "composite particle".) having a ratio between a long-term residual amount and a short-term residual amount (long-term residual amount/short-term residual amount) of less than 90%, wherein the long-term residual amount is an average of a contrast rate in cells after a lapse of 6 days and a contrast rate in cells of the same species after a lapse of 11 days and the short-term residual amount is a contrast rate in cells of the same species after a lapse of 2 days.

Herein, the "contrast rate" means the intensity of a signal detected in cells when the composite particle is used as a contrast agent. For example, the contrast rate can correspond to saturated magnetic susceptibility in a case where the composite particle is a contrast agent for MRI, the contrast rate can correspond to luminosity or brightness (X-ray absorbance) of the composite particle in an image taken in a case where the composite particle is a contrast agent for taking an image by X-ray, the contrast rate can correspond to the intensity of radiation in a case where the composite particle is a contrast agent of a contrast agent particle for positron emission tomography (PET), the contrast rate can correspond to the intensity of fluorescence in a case where the composite particle is a contrast agent for taking an image by fluorescence, and the contrast rate can correspond to ultrasonic intensity in a case where the composite particle is a contrast agent for photoacoustically taking an image.

The cells for use in measurements of the long-term residual rate and the short-term residual rate may be any cell as long as cells of the same species are used for such measurements, and are preferably cells derived from a readily available cell strain, more preferably adherent animal cells. The animal cells may be cells derived from any animal, and for example, any cell derived from human, pig, dog, mouse, and the like can be used. Examples of the adherent animal cell include cells high in adhesiveness to a culture vessel, such as primary cultured cell and established cell line, and examples include primary cultured cells such as epidermal keratinocytes, vascular endothelial cells, fibroblasts, osteoblasts and hepatocytes, and stem cells or progenitor cells, including embryonic stem cells, induced pluripotent stem cells, mesenchymal stem cells, adipose progenitor cells, and hepatic stem cells. The cells may be any cell into which a foreign gene is inserted before culturing, or any cell stimulated or processed in advance by a stimulation factor such as an antibody and a ligand.

### 1. Composite particle

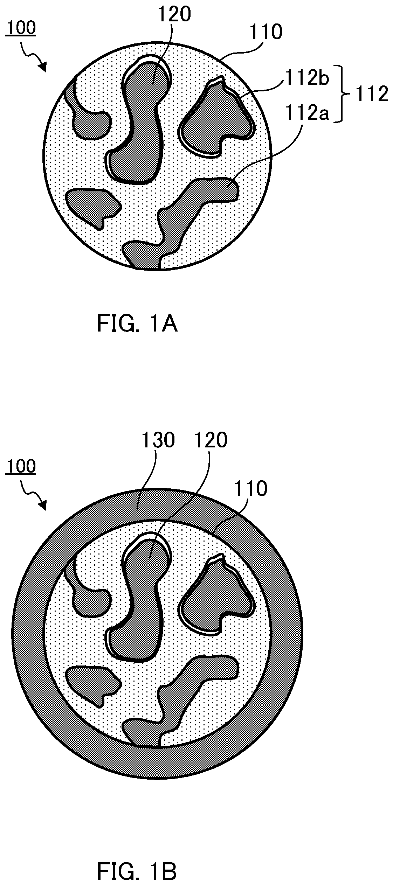

In one embodiment of the present invention, composite particle 100 includes contrast agent particle 110, and degradation accelerator 120 that accelerates degradation of contrast agent particle 110, as illustrated in FIG. 1A. Contrast agent particle 110 has pore 112 therein, and degradation accelerator 120 is present in pore 112 and is encapsulated in contrast agent particle 110. As illustrated in FIG. 1B, an outer edge of contrast agent particle 110 in composite particle 100 may be further covered with degradation accelerator 130. Herein, degradation accelerator 130 with which the particle is covered may be formed from a substance the same as or different from that of degradation accelerator 120 contained.

Composite particle 100 includes contrast agent particle 110. Contrast agent particle 110 is a particle that includes a substance having a contrast effect and is a particle that includes a substance whose presence is displayed in an image in a highlighted manner due to displaying of the substance in an image taken, at a different contrast or brightness from that of a living body substance in taking of an image of the inside of cells.

Examples of contrast agent particle 110 include known contrast agent particles such as a contrast agent particle for MRI, a contrast agent particle for taking an image by X-ray, a contrast agent particle for positron emission tomography (PET), a contrast agent particle for taking an image by fluorescence, and a contrast agent particle for photoacoustically taking an image.

Examples of the substance having a contrast effect, which can be included in the contrast agent particle for MRI, include respective magnetic materials including gadolinium (Gd) and iron oxide (such as Fe₃O₄, γ-Fe₂O₃, and ferrite).

Examples of the substance having a contrast effect, which can be included in the contrast agent particle for taking an image by X-ray, include tungsten, platinum, tantalum, iridium, gold, barium sulfate, bismuth subcarbonate, bismuth trioxide, bismuth oxychloride, metrizamide, iopamidol, sodium iotalamate, sodium iodide, and meglumine.

Examples of the substance having a contrast effect, which can be included in the contrast agent particle for taking an image by fluorescence, include fluorescent dyes including fluorescein and indocyanine green, and fluorescent proteins such as a green fluorescent protein (GFP).

Examples of the substance having a contrast effect, which can be included in the contrast agent particle for photoacoustically taking an image, include a gold nanoparticle, a single-wall carbon nanotube (SWNT), indocyanine green, and methylene blue.

Contrast agent particle 110 is a porous particle having pore 112. Pore 112 may have any size and shape so that an amount of degradation accelerator 120, which enables degradation of composite particle 100 to be accelerated, can be present in contrast agent particle 110 (in pore 112).

The specific surface area and the porosity of contrast agent particle 110 are desirably higher from the viewpoints that degradation accelerator 120 is more contained in contrast agent particle 110 and that the rate of degradation of composite particle 100 after an image is taken is enhanced. In particular, in a case where an image is taken by MRI, the specific surface area and the porosity of contrast agent particle 110 are desirably higher also from the viewpoint that saturated magnetic susceptibility is more enhanced due to penetration of water (proton) into a gap generated by slight dissolution or degradation of degradation accelerator 120 in pore 112 before an image is taken. On the other hand, the specific surface area and the porosity of contrast agent particle 110 are desirably not too high from the viewpoint that the contrast effect is inhibited from being deteriorated due to excess degradation of composite particle 100 administered, before an image is taken.

The specific surface area of contrast agent particle 110 is preferably 144 cm²/g or more and 11500 cm²/g or less, more preferably 400 cm²/g or more and 5500 cm²/g or less, further preferably 576 cm²/g or more and 5500 cm²/g or less from the above viewpoints.

The porosity of contrast agent particle 110 is preferably 10% or more and 90% or less, more preferably 20% or more and 80% or less, further preferably 30% or more and 70% or less from the above viewpoints.

The specific surface area and the porosity of contrast agent particle 110 are not limited to be within the respective ranges, and can be arbitrarily defined depending on, for example, the material of the contrast agent particle and the type of an observation object (depending on an enzyme that can be secreted by cells as an observation object, the pH in the cells (in particular, in a lysosome), and the like) as long as contrast agent particle 110 is easily degraded after an image is taken and composite particle 100 administered is not excessively degraded before an image is taken.

The specific surface area of contrast agent particle 110 can correspond to a value obtained by dividing the apparent surface area of contrast agent particle 110 by the product of the apparent volume and density of contrast agent particle 110. The apparent surface area of contrast agent particle 110 can be determined by determining the surface area of contrast agent particle 110, presumed from an outline of one contrast agent particle 110 based on a cross-sectional image of contrast agent particle 110 taken by a scanning electron microscope (SEM), with respect to a plurality of (for example, 20) contrast agent particles 110, and calculating the average of the surface areas determined, of such contrast agent particles 110. The apparent volume of contrast agent particle 110 can be determined by calculating the average particle size of contrast agent particle 110 from the weighted average of the average of a longer diameter and a shorter diameter of one contrast agent particle 110, based on the cross-sectional image of contrast agent particle 110 taken by SEM, determining the volume of contrast agent particle 110, calculated from the average particle size under the assumption that contrast agent particle 110 has a spherical shape, with respect to a plurality of (for example, 20) contrast agent particles 110, and calculating the average of the volumes determined, of such contrast agent particles 110. The density of contrast agent particle 110 can be a known density of the material of contrast agent particle 110, identified according to an ICP emission spectrochemical analysis method.

The porosity of contrast agent particle 110 can be a value (value obtained by cubing the square root of the area ratio) which is obtained by converting the area ratio of contrast agent particle 110 and pore 112 into the volume ratio thereof in the cross-sectional image of contrast agent particle 110 taken by SEM.

Herein, pore 112 may be fully filled with degradation accelerator 120 as in pore 112a illustrated in FIG. 1A, or may have a region in which degradation accelerator 120 is not partially present, as in pore 112b illustrated in FIG. 1A.

Degradation accelerator 120 is a substance that accelerates dissolution or degradation of the substance having a contrast effect to thereby accelerate degradation of composite particle 100. Degradation accelerator 120 can be a substance that can be more rapidly dissolved or degraded than contrast agent particle 110 in an intercellular environment to thereby accelerate degradation of contrast agent particle 110 and accelerate dissolution or degradation of the substance having a contrast effect.

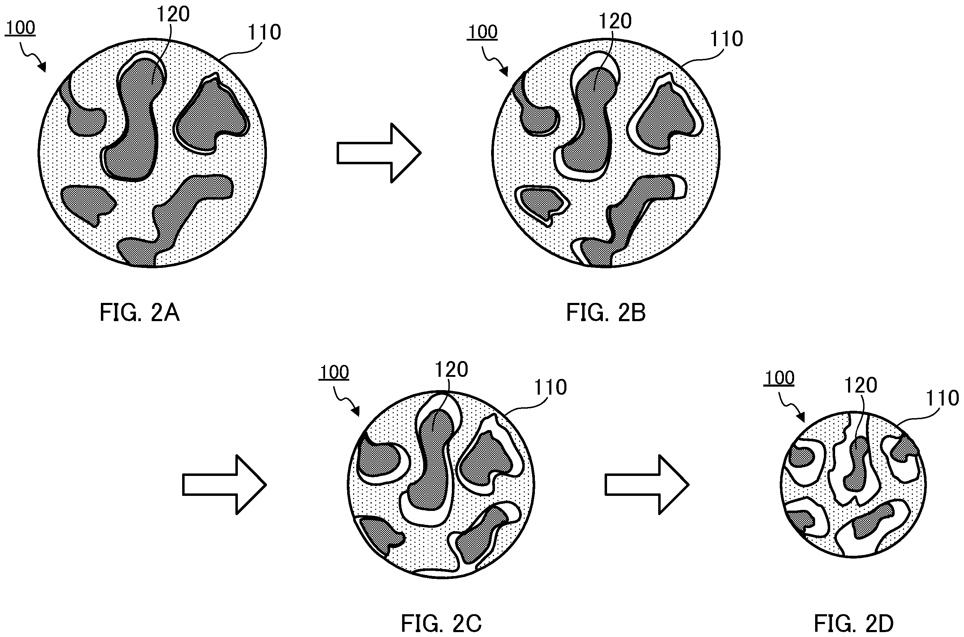

As illustrated in FIGS. 2A to 2D, when composite particle 100 (FIG. 2A) is administered into cells, degradation accelerator 120 included in composite particle 100 is preferentially dissolved or degraded due to the action of an enzyme in the cells or the action due to an acidic (low-pH) environment of lysosome or the like (FIG. 2B). Thus, the surface area of contrast agent particle 110 increases, thereby allowing also contrast agent particle 110 to be easily dissolved or degraded, and/or allowing the periphery of contrast agent particle 110 to be peeled at the same time as partial peeling of degradation accelerator 120, also resulting in acceleration of dissolution or degradation of contrast agent particle 110 (FIG. 2C). Thus, it is considered that composite particle 100 is acceleratedly dissolved or degraded after a lapse of a certain time from administration (after an image is taken) (FIG. 2D).

On the other hand, degradation accelerator 120 is present in pore 112 immediately after administration, and thus an enzyme in the cells and an acidic intracellular fluid hardly penetrate in pore 112, resulting in suppression of dissolution or degradation of the composite particle.

It is considered that composite particle 100 is characterized by being hardly dissolved or degraded immediately after administration and being acceleratedly dissolved or degraded after a lapse of a certain time from administration, due to the above-described actions. Thus, contrast agent particle 110 included in composite particle 100 has a size sufficient for having a favorable contrast effect and is favorable in contrast effect until an image is taken after administration, and thereafter is rapidly dissolved or degraded and removed from cells.

Thus, composite particle 100 can be characterized in that, when the rate of decrease per unit time in the concentration of composite particle 100 included in living cells after administration to the living cells is measured, the rate of decrease at a shorter elapsed time after administration is lower than the rate of decrease at a longer elapsed time after administration. Such a characteristic indicates that, while the speed of dissolution or degradation of composite particle 100 is low after administration to living cells (the rate of decrease is low), the speed of dissolution or degradation increases according to a lapse of time (the rate of decrease is increased). The shorter elapsed time after administration and the longer elapsed time after administration may be periods not overlapping with each other, and can be arbitrarily determined. For example, the rate of decrease per day in the concentration of composite particle 100 until a lapse of 2 days from administration can be defined as the "rate of decrease at a shorter elapsed time after administration", and the rate of decrease per day in the concentration of composite particle 100 until a lapse of 11 days after a lapse of 6 days from administration can be defined as the "rate of decrease at a longer elapsed time after administration". The concentration of composite particle 100 in living cells can be the concentration of the substance having a contrast effect in living cells. A composite particle satisfying the above properties can be produced by varying the type or the amount of degradation accelerator 120.

For example, when contrast agent particle 110 is a particle including an atom of a metal element, degradation accelerator 120 can be a compound including an atom of any metal element (hereinafter, simply referred to as "easily ionizable atom".) that is higher in ionization tendency than the metal element, or a compound including a chelating agent (hereinafter, simply referred to as "chelating agent".) capable of being coordinately bound to the metal atom. Alternatively, degradation accelerator 120 can be a biodegradable substance that is to be degraded by an enzyme or due to the pH, in cells. Herein, degradation accelerator 120 may include water other than the respective substances.

Degradation accelerator 120 may be the compound or substance singly, or may be a combination of the compounds or substances. In particular, degradation accelerator 120 easily allows iron oxide particles to be kept in a dispersed state, and is preferably a combination of a compound including the easily ionizable atom, or the chelating agent, with the biodegradable substance.

For example, when contrast agent particle 110 is a particle including a Fe atom, degradation accelerator 120 can be a compound including, as the easily ionizable atom, an atom of a metal element higher in ionization tendency than Fe, such as Mg, Na, Ca, K, Al, and Zn. The compound including such an atom can be, for example, a salt including the element, or an oxide or nitride of the element. In particular, a salt is preferable from the viewpoint that the degradation accelerator is more easily dissolved. Such a compound easily allows composite particle 100 to be kept in a dispersed state, and thus not only more enhances the contrast rate of composite particle 100, but also further accelerates degradation of composite particle 100 by degradation accelerator 120.

The chelating agent can be appropriately selected depending on the type of the metal element included in the contrast agent particle. Examples of the chelating agent include ethylenediaminetetraacetic acid (EDTA), nitrilotriacetic acid (NTA), hydroxyethyliminodiacetic acid (HIDA), hydroxyethylethylenediaminetriacetic acid (HEDTA), diethylenetriaminepentaacetic acid (DTPA), triethylenetetraaminehexaacetic acid (TTHA), dicarboxymethylglutamic acid (GLDA), dihydroxyethylglycine (DHEG), 1,3-propanediaminetetraacetic acid (PDTA), glycol ether diaminetetraacetic acid (GEDTA), 1,3-diamino-2-hydroxypropanetetraacetic acid (DPTA-OH), hydroxyethanediphosphonic acid (HEDP), nitrilotrismethylenephosphonic acid (NTMP), phosphonobutanetricarboxylic acid (PBTC), citric acid, and ascorbic acid.

The biodegradable substance may be a substance including a material that is hydrolyzed in cells, degraded by an enzyme secreted in cells, or dissolved or degraded in an acidic environment in a lysosome or the like in cells. For example, the biodegradable substance is preferably a water-soluble compound, more preferably a hydrophilic polymer.

The biodegradable substance as the hydrophilic polymer is preferably a hydrogel from the viewpoint that degradation accelerator 120 is easily dissolved or degraded in cells. The hydrogel means any gel that can be formed with water as a solvent. The hydrogel typically includes a hydrophilic polymer that forms a network structure, and water incorporated in the network structure.

Examples of the hydrogel include polysaccharides including chitin, chitosan, hyaluronic acid, alginic acid, agarose, carboxymethyl cellulose (CMC), starch, and pectin, proteins including gelatin, collagen, fibrin, and albumin, polyamino acids including poly-γ-glutamic acid, poly-L-lysine, and polyarginine, and synthetic polymers including acrylamide, silicone, polyvinyl alcohol, polyethylene oxide, and polyvinylpyrrolidone.

In particular, polysaccharides, proteins, and polyamino acids are preferable from the viewpoint that incorporation of composite particle 100 into living cells is facilitated by the cells by themselves to thereby enable the living cells to be inhibited from being damaged in such incorporation, and polysaccharides and proteins are more preferable and gelatin is further preferable in terms of availability or ease of production.

The gelatin is, specifically, a protein that includes 300 or more glycine residues among 1000 amino acid residues and includes both alanine and proline, when analyzed by an amino acid analyzer. The gelatin may be any known gelatin that is obtained by denaturing collagen derived from cattle bone, cattle skin, pig skin, pig tendon, fish scales, and fish meat. Gelatin has been previously used for foods and for medical purposes, and its intake into the body is hardly harmful to the human body.

Degradation accelerator 120 is expected to also have the effect of trapping a substance having an contrast effect, which is dissolved or degraded, to inhibit the substance having an contrast effect from being deposited and precipitated in cells, thereby allowing the substance having an contrast effect to be easily excreted. For example, when the substance having a contrast effect is Fe, a carboxyl group or the like included in the hydrophilic polymer (form R-COOFe) can trap a Fe ion dissolved to excrete Fe together with the hydrophilic polymer out of cells.

The weight average molecular weight of the hydrophilic polymer is preferably 1,000 or more and 100,000 or less. The weight average molecular weight can be, for example, a molecular weight in terms of styrene, obtained by gel permeation chromatography (GPC), or the weight average molecular weight of the gelatin can be a value measured according to the PAGI Method Ver. 10 (2006).

The hydrophilic polymer may be crosslinked. Such crosslinking may be crosslinking with a crosslinking agent, or self-crosslinking made without any crosslinking agent.

The crosslinking agent may be, for example, a compound having a plurality of functional groups each forming a chemical bond with a hydroxyl group, a carboxyl group, an amino group, a thiol group, an imidazole group, or the like. Examples of such a crosslinking agent include glutaraldehyde, water-soluble carbodiimides including 1-ethyl-3-(3-dimethylaminopropyl)carbodiimide hydrochloride (EDC) and 1-cyclohexyl-3-(2-morpholinoethyl)carbodiimide-metho-p-toluenesulfonate (CMC), and compounds having two or more epoxy groups, including ethylene glycol diglycidyl ether, polyethylene glycol diglycidyl ether, polyglycol polyglycidyl ether, and glycol polyglycidyl ether, and propylene oxide. In particular, glutaraldehyde and EDC are preferable, and glutaraldehyde is more preferable, from the viewpoint of a more enhancement in reactivity.

Examples of the self-crosslinking include crosslinking made by application of heat, or irradiation with electron beam or ultraviolet light.

The amount of water included in degradation accelerator 120 is not particularly limited, and is preferably 1mass% or more and 99mass% or less, more preferably 10mass% or more and 90mass% or less, further preferably 15mass% or more and 80mass% or less based on the total mass of degradation accelerator 120, after a swelling treatment.

Herein, "after a swelling treatment" means a treatment of immersion of dried composite particle 100 in water at 40°C under an atmospheric pressure for 60 minutes. Herein, dried composite particle 100 means composite particle 100 after standing in air at 80°C for 24 hours.

In a case where degradation accelerator 120 in composite particle 100 is a compound including an easily ionizable atom, the amount of the compound including an easily ionizable atom based on the total mass of components other than water included in composite particle 100 is preferably 0.1mass% or more and 90mass% or less. The amount of the compound including an easily ionizable atom is 0.1mass% or more, thereby favorably accelerating degradation of contrast agent particle 110 due to dissolution or degradation of degradation accelerator 120. In particular, in a case where an image is taken by MRI, the amount of the compound including an easily ionizable atom is 0.1mass% or more, thereby enabling a more enhancement in saturated magnetic susceptibility due to penetration of water (proton) into a gap generated by slight dissolution of such an easily ionizable atom in pore 112 before an image is taken. The amount of the compound including an easily ionizable atom is 90mass% or less, thereby allowing the contrast effect of composite particle 100 to be sufficiently exerted because composite particle 100 includes a sufficient amount of contrast agent particle 110. The amount of the compound including an easily ionizable atom based on the total mass of components other than water included in composite particle 100 is more preferably 0.1mass% or more and 25mass% or less, further preferably 0.5mass% or more and 20mass% or less from the above viewpoint. In a case where contrast agent particle 110 is further covered with degradation accelerator 120, the amount of the compound including an easily ionizable atom corresponds to a value including the amount of any easily ionizable atom forming degradation accelerator 120 with which contrast agent particle 110 is covered.

The total mass of the chelating agent included in composite particle 100 is preferably 0.1mass% or more and 80mass% or less, more preferably 0.1mass% or more and 25mass% or less, further preferably 0.5mass% or more and 20mass% or less based on the total mass of any component(s) other than water included in composite particle 100. In a case where contrast agent particle 110 is further covered with degradation accelerator 120, the amount of the chelating agent corresponds to a value including the amount of any easily ionizable atom forming degradation accelerator 120 with which contrast agent particle 110 is covered.

In a case where degradation accelerator 120 in composite particle 100 is the hydrophilic polymer, the amount of the hydrophilic polymer based on the total mass of components other than water included in composite particle 100 is preferably 1.0mass% or more and 95mass% or less. The amount of the hydrophilic polymer is 1.0mass% or more, thereby favorably accelerating degradation of contrast agent particle 110 due to dissolution or degradation of degradation accelerator 120. In particular, in a case where an image is taken by MRI, the amount of the hydrophilic polymer is 5mass% or more, thereby enabling a more enhancement in saturated magnetic susceptibility due to penetration of water (proton) into a gap generated by slight dissolution or degradation of the hydrophilic polymer in pore 112 before an image is taken. The amount of the hydrophilic polymer is 95mass% or less, thereby allowing the contrast effect of composite particle 100 to be sufficiently exerted because composite particle 100 includes a sufficient amount of contrast agent particle 110. The amount of the hydrophilic polymer based on the total mass of components other than water included in composite particle 100 is preferably 1.0mass% or more and 95mass% or less, more preferably 5.0mass% or more and 95mass% or less, more preferably 25mass% or more and 95mass% or less, further preferably 50mass% or more and 95mass% or less, from the above viewpoint. In a case where contrast agent particle 110 is further covered with degradation accelerator 120, the amount of the hydrophilic polymer corresponds to a value including the amount of any hydrophilic polymer forming degradation accelerator 120 with which contrast agent particle 110 is covered.

The total mass of components other than water included in composite particle 100 may be any mass of dried composite particle 100. The total mass of the easily ionizable atom included in composite particle 100 can be a value measured according to an ICP emission spectrochemical analysis method. In a case where composite particle 100 including a particle having pore 112 and other substance contained in pore 112 is observed in an image taken by SEM, and a metal element that can serve as a contrast agent and any metal element higher in ionization tendency than the metal element are identified according to an ICP emission spectrochemical analysis method, it can be presumed that the former metal element corresponds to an element forming contrast agent particle 110 and the latter metal element corresponds to an element as the easily ionizable atom forming degradation accelerator 120. Here, the volumes of the particle, and the substance in pore 112, presumed with the image taken by SEM, may be each compared with the volume of each element, which is calculated from the mass of such each element measured according to an ICP emission spectrochemical analysis method, and the specific gravity of such each element, thereby confirming the following: the particle and the substance, and the element correspond to an element forming contrast agent particle 110 and an element forming degradation accelerator 120, respectively.

The total mass of the biodegradable substance or the chelating agent included in composite particle 100 can be a value measured according to a known method such as FT-IR.

For example, the amount of the biodegradable substance may be calculated from the height or the area of a peak corresponding to a functional group or a structure (for example, COOH) expected to be derived from the biodegradable substance, based on a spectrum obtained by subjecting composite particle 100 (or a precipitate recovered by centrifugation of a slurry including composite particle 100 at 13000 rpm for 20 minutes) to FT-IR measurement in conditions exemplified below.

Measurement apparatus: Fourier transform infrared spectrophotometer Nicolet 380 (manufactured by Thermo Fisher Scientific)
FT-IR measurement condition: single reflection ATR method
Detection range: 4,000 to 700 cm⁻¹
Number of scans: 32 times
Window material used: Ge
Resolution: 4
Beam splitter: KBr
Gain: 2
Light source: IR
Detector: DTGS KBr

Composite particle 100 may include the substance having a contrast effect in an amount that allows a sufficient contrast rate to be obtained. For example, when contrast agent particle 110 is a contrast agent particle for MRI, the amount of the substance having an contrast effect is an amount so that the saturated magnetic susceptibility of composite particle 100 at a water content of 0.01mass% or more and 10mass% or less is preferably 1.0 emu/g or more and 150 emu/g or less, more preferably 30 emu/g or more and 140 emu/g or less.

Composite particle 100 is preferably a particle having an average particle size of 2.0 nm or more and 1500 nm or less from the viewpoint that incorporation into cells is facilitated by the cells by themselves to thereby inhibit living cells from being damaged in such incorporation.

Composite particle 100 having an average particle size of 2.0 nm or more easily allows the substance having a contrast effect to be more included in the particle. The particle size of composite particle 100 is preferably 10 nm or more, more preferably 20 nm or more from the above viewpoint.

Composite particle 100 having an average particle size of 1,500 nm or less is easily incorporated into living cells by their own action. The reason for this is considered because composite particle 100 having a particle size of 1,500 nm or less is hardly recognized as a foreign substance by living cells and is easily incorporated into cells by the action of endocytosis or the like. The particle size of composite particle 100 is preferably 1,000 nm or less, more preferably 800 nm or less, further preferably 600 nm or less from the above viewpoint.

The average particle size of composite particle 100 is preferably smaller, in particular, from the viewpoint the ratio of the surface area to the volume of composite particle 100 is increased to thereby allow composite particle 100 to be easily dissolved, and is preferably 2.0 nm or more and 500 nm or less, more preferably 2.0 nm or more and 400 nm or less, further preferably 2.0 nm or more and 300 nm or less.

The aspect ratio of composite particle 100 dried is preferably 1.0 or more and 1.4 or less. In a case where the aspect ratio is 1.4 or less, it is considered that composite particle 100 has a shape closer to a spherical shape to thereby allow composite particle 100 and cells in a solution including composite particle 100 and the cells to be contacted on a contact surface more uniform in shape and size, thereby hardly causing the difference in ease of incorporation of composite particle 100 into the cells. Thus, it is considered that composite particle 100 having the aspect ratio is more easily controlled in the amount of composite particle 100 incorporated into the cell, and the amount of the cell that incorporates composite particle 100. The aspect ratio of composite particle 100 can be a value determined by dividing a longer diameter of dried composite particle 100 by a shorter diameter of dried composite particle 100.

The average particle size of composite particle 100 can be determined by determining the particle size of composite particle 100, calculated as the weighted average of the average of a longer diameter and a shorter diameter of one composite particle 100 based on a cross-sectional image of composite particle 100 taken by SEM, with respect to a plurality of (for example, 20) composite particles 100, and calculating the average of the particle sizes determined, of such composite particles 100. The aspect ratio of composite particle 100 can be determined by determining the aspect ratio of composite particle 100, calculated as the ratio (shorter diameter/longer diameter) of a shorter diameter and a longer diameter of one composite particle 100 based on the cross-sectional image of composite particle 100 taken by SEM, with respect to a plurality of (for example, 20) composite particles 100, and calculating the average of the aspect ratios determined, of such composite particles 100.

Composite particle 100 may further include various drugs. Examples of such a drug include proteins having pharmaceutical activity, plasmids, aptamers, antisense nucleic acids, ribozymes, nucleic acids for use in pharmaceutical application, including tRNA, snRNA, siRNA, shRNA, ncRNA, and condensed DNA, and antigens for use in pharmaceutical application.

Examples of the protein having pharmaceutical activity include steroids, nonsteroidal anti-inflammatory drugs (NSAIDs), vitamin A (retinoid), vitamin D3 and vitamin D3 analogs, antibiotic substances, antiviral drugs, and antibacterial drugs.

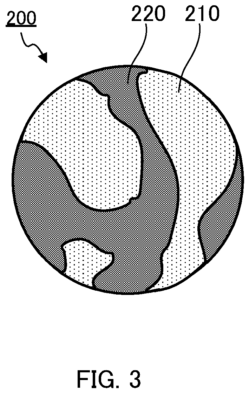

In another embodiment of the present invention, as illustrated in FIG. 3, composite particle 200 may be a particle where both substance 210 having a contrast effect and degradation accelerator 220 form no clear particle singly and are present in a mutually mixed manner. Herein, "containing" means a configuration where the degradation accelerator is located more inwardly than the substance having a contrast effect.

In all the embodiments, an outer edge of composite particle 100 or composite particle 200 may be covered with the degradation accelerator. Thus, the long-term residual rate and the short-term residual rate can be each adjusted, respectively.

### 2. Method for producing composite particle

The composite particle can be produced by preparing a liquid including the substance having a contrast effect and the degradation accelerator (hereinafter, simply referred to as "material solution".), and forming a particle of the substance having a contrast effect in the material solution. Such particle formation can be performed according to, for example, a method where a material solution droplets are discharged into an atmosphere in a heating tube or a drying chamber and dried (in-air dropping method), a method where a material solution droplets are discharged into a hydrophobic solvent and dispersed (in-liquid dropping method), or a method where a material solution is formed into an emulsion and a fine droplet including gelatin is dispersed (in-liquid dispersion method).

The degradation accelerator can also be further provided to the composite particle produced according to the above method or the like, thereby providing a composite particle further covered with the degradation accelerator.

The material solution may be obtained by mixing the substance having a contrast effect and the degradation accelerator, or may be formed into a slurry including the degradation accelerator and the substance having a contrast effect by synthesis of the substance having a contrast effect in a solution including the degradation accelerator. The slurry can be warmed to thereby form the substance having a contrast effect into a particle, thereby providing the composite particle. In a case where the composite particle further covered with the degradation accelerator is produced, a phase separation inducer may be added to the slurry. The composite particle produced can be purified and separated from the slurry.

For example, in a case where a composite particle including Fe₃O₄ as the substance having a contrast effect is produced, an aqueous solution including FeCl₃•6H₂O and FeCl₂•4H₂O as raw materials of Fe₃O₄ and the degradation accelerator is prepared, and the pH of the solution is adjusted to 7 or more by addition of an alkaline solution (for example, solution of NaOH, NH₃, KOH or the like) thereto, to thereby synthesize Fe₃O₄, thereby providing a slurry where particulate Fe₃O₄ containing the degradation accelerator is uniformly dispersed.

The resulting slurry can be warmed to 50°C or more and 80°C or less, to thereby form Fe₃O₄ into a porous particle, thereby providing a composite particle including the degradation accelerator in a pore. Furthermore, a phase separation inducer can also be added to a slurry including the resulting composite particle to thereby generate coacervation of the degradation accelerator (in particular, hydrophilic polymer such as gelatin), thereby providing a composite particle further covered with the degradation accelerator.

The phase separation inducer is not particularly limited as long as the inducer is a component that can impart phase separation of the degradation accelerator. Examples of the phase separation inducer include alcohols including ethanol, 1-propanol, 2-propanol, and 1-butanol, and organic solvents including acetone.

In a case where the phase separation inducer is added to the slurry, the concentration of the degradation accelerator in the slurry can be adjusted and thus the average particle size of the resulting composite particle can be adjusted. In other words, there is the following tendency: as the concentration of the degradation accelerator in the slurry is higher, the average particle size of the resulting composite particle is larger, and as the concentration of the degradation accelerator in the slurry is lower, the average particle size of the resulting composite particle is smaller. In a case where MgCl₂ as the easily ionizable atom is used as the degradation accelerator, there is particularly the following tendency: as the concentration of the degradation accelerator in the slurry is higher, the average particle size of the resulting composite particle is larger.

The amount of the degradation accelerator in the material solution is preferably an amount so that the amount of the easily ionizable atom, the chelating agent or the hydrophilic polymer based on the total mass of components other than water included in the resulting composite particle is within the above range. For example, the amount of the degradation accelerator is preferably an amount so that the amount of the easily ionizable atom based on the total mass of a powder obtained by freeze-drying the material solution (or the slurry) is 0.1mass% or more and 80mass% or less, an amount so that the amount of the chelating agent based on the total mass of the powder obtained by freeze-drying is 0.1mass% or more and 80mass% or less, or an amount so that the amount of the hydrophilic polymer based on the total mass of the powder obtained by freeze-drying is 5mass% or more and 80mass% or less.

The amount of the substance having a contrast effect in the material solution may be any amount that enables the resulting composite particle to include the substance having an contrast effect in an amount that allows a sufficient contrast rate to be obtained, and, for example, when the contrast agent particle is a contrast agent particle for MRI, the amount of the substance having an contrast effect may be any amount so that the saturated magnetic susceptibility of the composite particle is 1.0 emu/g or more and 150 emu/g or less, preferably 30 emu/g or more and 140 emu/g or less.

### 3. Cell including composite particle

Still another embodiment of the present invention relates to a cell having the composite particle in the interior of a cell membrane (hereinafter, simply referred to as "composite particle-encapsulating cell".).

The phrase "having the composite particle in the interior of a cell membrane" means that the composite particle is found in the interior of a cell membrane in an image of a cell, taken by TEM.

Examples of the cells capable of including the composite particle in the interior of a cell membrane include known cells including cells derived from biological samples or specimens extracted from various organs such as the bone marrow, heart, lung, liver, kidney, pancreas, spleen, intestinal tract, small intestine, cardiac valve, skin, blood vessel, cornea, eyeball, dura mater, bone, trachea, and auditory ossicle, commercially available established cell line, and stem cells including skin stem cell, epidermal keratinocyte stem cell, retinal stem cell, retinal epithelial stem cell, cartilage stem cell, hair follicle stem cell, muscle stem cell, osteoprogenitor stem cell, adipose progenitor cell, hematopoietic stem cell, nerve stem cell, hepatic stem cell, pancreatic stem cell, ectodermal stem cell, mesodermal stem cell, endodermal stem cell, mesenchymal stem cell, ES cell, and iPS cell, and cells differentiated from such cells.

The composite particle-encapsulating cell may be a single cell or may have a cell structure including a plurality of such composite particle-encapsulating cells.

Examples of the cell structure include a cell sheet where a plurality of such composite particle-encapsulating cells are two-dimensionally cultured, and a spheroid (cell mass) where a plurality of such composite particle-encapsulating cells are three-dimensionally cultured. The cell structure may be a structure where a plurality of such composite particle-encapsulating cells are each covered with a film including a polymer, or may be a structure where the surface of a steric structure (bead) having any shape, including a polymer, is covered with a plurality of such composite particle-encapsulating cells. Examples of the polymer include laminin, proteoglycan, fibrin, matrigel, chitosan gel, polyethylene glycol, gelatin, and alginic acid, and a polymer capable of forming an extracellular matrix is preferable.

The composite particle-encapsulating cell or the cell structure may be a mixed dispersion with the polymer. A dispersion medium of the mixed dispersion can be a cell culture solution. The cell culture solution may be a known buffer or saline, and can be, for example, a Hank's balanced salt solution (HBSS), 4-(2-hydroxyethyl)-1-piperazineethanesulfonic acid (HEPES), and other known phosphate buffered saline (PBS).

The composite particle-encapsulating cell can be cultured in a structure or mixed dispersion including the composite particle-encapsulating cell or the cell structure and the polymer, thereby allowing a spheroid, a cell sheet or the like formed, to coalesce into a larger cell population, thereby providing a tissue-like three-dimensional cell culture.

The cell structure or the mixed dispersion may include only one composite particle-encapsulating cell, a plurality of composite particle-encapsulating cells, or other cell including no composite particle. For example, cells included in a cell structure or a mixed dispersion including a composite particle-encapsulating cell for tissue formation and cells for vascular formation can be cultured to thereby provide an organ-like three-dimensional cell structure having a blood vessel.

Cells to be transplanted into a patient in cell regenerative medicine and cell structure including the cell, in particular, a stem cell or a cell differentiated from a stem cell, or a cell taken from a patient and subjected to therapy with administration or the like of a drug, and a cell structure including the cell can include a composite particle, and thus can be observed about whether or not a composite particle-encapsulating cell (or cell structure) is colonized in a transplantation site, without reoperation such as an incision, by taking an image of the transplantation site after transplantation into the patient. Thus, it is considered that such a composite particle-encapsulating cell and a cell structure including such a composite particle-encapsulating cell can reduce any physical, mental, financial, and temporal burden on a patient receiving therapy of regenerative medicine and improve quality of life (QOL) of the patient.

It is also considered that high-activity cells more rapidly degrade and easily excrete a composite particle and high-activity cells more slowly degrade and excrete a composite particle. Thus, the activity of cells can also be non-destructively examined by measurement of the contrast rate in the composite particle-encapsulating cell over time.

### 4. Method for producing cell including composite particle

The composite particle-encapsulating cell can be produced by introducing a composite particle into the cell. Examples of the method for introducing a composite particle into the cell include a method where a composite particle and the cell are added into a liquid and incorporation by cell's own action such as incorporation due to endocytosis is made, and a method where introduction is made by an external operation. Examples of such a method for incorporation by cell's own action include a method where a composite particle and the cells are stirred in a liquid, and a method where cells are cultured in a cell culture solution including a composite particle. Such a composite particle is high in incorporation efficiency by the cells by themselves, and thus there is no particular need for any operation that forms a composite with other component in order to accelerate incorporation into the cells. Examples of the method where introduction is made by an external operation include an electroporation method and a microinjection method. In particular, such a method for incorporation by cell's own action is preferable, and such a method for incorporation into the cell with no formation of any composite is more preferable, from the viewpoint that the activity of the cell is hardly decreased in introduction of a composite particle.

The above cell culture solution can be used as the liquid to which a composite particle and the cell are to be added.

The temperature of the cell culture solution in the above stirring is preferably 15°C or more and 50°C or less, more preferably 35°C or more and 45°C or less from the viewpoint that the cell activity is increased to allow a composite particle to be easily incorporated into the cell by cell's own action.

In a case where a composite particle is introduced in the interior of a cell membrane by cell's own action, such introduction may be accelerated by, for example, shaking a cell culture solution including a composite particle and the cells.

It is considered that, in a case where a composite particle is introduced by cell's own action, high-activity cells more easily incorporate a composite particle and low-activity cells hardly incorporates a composite particle. Thus, the cell activity can also be non-destructively examined by adding a composite particle and the cells to a liquid and, if necessary, shaking them, and thereafter measuring the contrast rate of the inside of the cells.

### Examples

Hereinafter, specific Examples of the present invention will be described. It is noted that the scope of the present invention is not construed as being limited by these Examples.

### 1. Production of particle

After FeCl₂·4H₂O, FeCl₃·6H₂O, each degradation accelerator shown in Table 1 and Table 2, and an aqueous 28% ammonia solution were each added in proportions shown in Table 1 and Table 2, into 200 ml of ultrapure water, the resultant was stirred in a water bath warmed to 70°C, for 20 minutes, thereby producing a slurry including a particle where each degradation accelerator was included in a contrast agent particle including iron oxide. The slurry produced was purified by using a PD-10 column manufactured by GE Healthcare Japan, and thus particle 1 to particle 14 and particle 16 to particle 19 were each obtained.

Furthermore, while 20 ml of the slurry not purified was stirred at 50°C, 100 ml of acetone was dropped and thereafter 3.5 ml of 25% glutaraldehyde was added. Furthermore, glycine was added to stop a reaction of an unreacted crosslinking agent, and a slurry including a particle further covered with gelatin was produced. The slurry produced was loaded into a centrifuge, and centrifuged at 13,000 rpm for 30 minutes. A precipitate was recovered and dispersed in water, thereby providing particle 15.

After FeCl₂·4H₂O, FeCl₃·6H₂O, and 28% ammonia water were each added in proportions shown in Table 2 into 200 ml of ultrapure water, the resultant was stirred in a water bath warmed to 60°C, for 30 minutes, thereby producing a slurry. The slurry produced was purified by using a PD-10 column manufactured by GE Healthcare Japan. Into the slurry purified was added 0.05 g of MgCl₂·6H₂O, and stirred overnight, thereby providing particle 20.

After FeCl₂·4H₂O, FeCl₃·6H₂O, and 28% ammonia water were each added in proportions shown in Table 2 into 200 ml of ultrapure water, the resultant was stirred in a water bath warmed to 60°C, for 30 minutes, thereby producing a slurry. The slurry produced was purified by using a PD-10 column manufactured by GE Healthcare Japan. Into the slurry purified was added 1 g of gelatin, and stirred overnight, thereby providing particle 21.

After FeCl₂·H₂O, FeCl₃·6H₂O, and 28% ammonia water were each added in proportions shown in Table 2 into 200 ml of ultrapure water, the resultant was stirred in a water bath warmed to 60°C, for 30 minutes, thereby producing a slurry and providing particle 22.

A cross-sectional image of such each particle, taken by SEM, was used to determine the particle size of the particle, calculated as the weighted average of the average of a longer diameter and a shorter diameter of such one particle, with respect to such 20 particles, and the average of the particle sizes of such 20 particle was determined, and defined as the average particle size of such each particle.

The amount of each material used in production of particle 1 to particle 22 and the average particle size with respect to the particle size of each of the resulting particles are shown in Table 1 and Table 2. In Table 1 and Table 2, "PVA" represents polyvinyl alcohol, "metal salt" represents MgCl₂·6H₂O, and "28%NH₄" represents an aqueous 28% ammonia solution, respectively. The "Externally added" described in the column of "Degradation accelerator" indicates no observation of any degradation accelerator contained in a contrast agent particle even in observation of the resulting particle with SEM because of covering with the degradation accelerator after production of the contrast agent particle, and the presence of the degradation accelerator only around the contrast agent particle. The number described in "Amount of degradation accelerator" indicates the amount of the degradation accelerator included in such each particle, calculated based on the ratio of materials used in production of such each particle.

**[Table 1]**

| | Material | | | | | | Particle | |
|---|---|---|---|---|---|---|---|---|
| | FeCl₂·4H₂O (g) | FeCl₃·6H₂O (g) | Degradation accelerator | | | 28%NH4 (ml) | Amount of degradation accelerator (mass%) | Average particle size (nm) |
| | | | Gelatin (g) | PVA (g) | Metal salt (g) | | | |
| Particle 1 | 0.75 | 1.8 | 0 | 0 | 0.003 | 45 | 0.4 | 120 |
| Particle 2 | 0.75 | 1.8 | 0 | 0 | 0.050 | 45 | 5.6 | 50 |
| Particle 3 | 0.75 | 1.8 | 0 | 0 | 0.060 | 45 | 6.7 | 30 |
| Particle 4 | 0.75 | 1.8 | 0 | 0 | 2.3 | 45 | 73.2 | 240 |
| Particle 5 | 0.75 | 1.8 | 0 | 0 | 7.1 | 45 | 89.4 | 310 |
| Particle 6 | 0.75 | 1.8 | 0.03 | 0 | 0 | 45 | 3.4 | 240 |
| Particle 7 | 0.75 | 1.8 | 0.2 | 0 | 0 | 45 | 19.2 | 150 |
| Particle 8 | 0.75 | 1.8 | 1.0 | 0 | 0 | 45 | 54.3 | 200 |
| Particle 9 | 0.75 | 1.8 | 2.0 | 0 | 0 | 45 | 70.4 | 120 |
| Particle 10 | 0.75 | 1.8 | 10 | 0 | 0 | 45 | 92.2 | 980 |

**[Table 2]**

| | Material | | | | | | Particle | |
|---|---|---|---|---|---|---|---|---|
| | FeCl₂·4H₂O (g) | FeCl₃·6H₂O (g) | Degradation accelerator | | | 28%NH₄ (ml) | Amount of degradation accelerator (mass%) | Average particle size (nm) |
| | | | Gelatin (g) | PVA (g) | Metal salt (g) | | | |
| Particle 11 | 0.75 | 1.8 | 1.0 | 0 | 0.05 | 45 | 55.5 | 75 |
| Particle 12 | 0.75 | 1.8 | 4.0 | 0 | 0.05 | 45 | 82.8 | 55 |
| Particle 13 | 0.75 | 1.8 | 10 | 0 | 0.05 | 45 | 92.3 | 155 |
| Particle 14 | 0.75 | 1.8 | 0 | 1.0 | 0 | 45 | 54.3 | 240 |
| Particle 15 | 0.75 | 1.8 | 10 | 0 | 0.05 | 45 | 92.3 | 240 |
| Particle 16 | 0.75 | 1.8 | 0.001 | 0 | 0 | 45 | 0.1 | 390 |
| Particle 17 | 0.75 | 1.8 | 15 | 0 | 0 | 45 | 94.7 | 550 |
| Particle 18 | 0.75 | 1.8 | 0 | 0 | 0.001 | 45 | 0.1 | 1600 |
| Particle 19 | 0.75 | 1.8 | 0 | 0 | 15 | 45 | 94.7 | 1200 |
| Particle 20 | 0.75 | 1.8 | 0 | 0 | 0.050 | 45 | 5.6 | 450 |
| Particle 21 | 0.75 | 1.8 | 1.0 | 0 | 0 | 45 | 54.3 | 360 |
| Particle 22 | 0.75 | 1.8 | 0 | 0 | 0 | 45 | 0.0 | 2400 |

### 2. Production of evaluation sample

A cell culture solution used was obtained by adding 50 ml of Fetal bovine serum to 500 ml of a cell culture solution MEM Alpha basic (IX) manufactured by Life Technologies. To 3 ml of the cell culture solution was added 1 mg of each of particle 1 to particle 22, mouse osteoblast-derived cells (MC3T3E1) were added at 1000 cells/ml, and the cell culture solution after addition of the cell was warmed at 40°C for 24 hours, thereby producing an evaluation sample.

### 3. Evaluation

### 3-1. Incorporation by cells

A portion of each cell dispersion was taken out from the evaluation sample, whether or not a particle incorporated in the interior of a cell membrane could be identified was observed according to the following procedure, and rating according to the following criteria was made.

### (Staining of cells and Fe)

To cells cultured was added 1 ml of 1% paraformaldehyde, to perform a cell immobilization treatment. Next, 1 ml of a Fe staining solution having the following composition was added to stain Fe. Furthermore, 1 ml of a nuclear staining solution having the following concentration adjusted was added to stain the cells.

### (Composition of Fe staining solution)

Equal volumes of the following two liquids were mixed, thereby preparing a Fe staining solution.
- 20volume% HCL (5-fold dilution of concentrated hydrochloric acid)
- Aqueous 10mass% K₄(Fe(CN₆)) solution (100 mg/ml)

### (Composition of nuclear staining solution)

5 parts by mass of ammonium sulfate and 0.1 parts by mass of Nuclear fast red were mixed with 100 parts by mass of distilled water, thereby preparing a nuclear staining solution.

### (Counting of number of cells incorporating Fe)

The cells stained were observed with an optical microscope, and ease of incorporation of each particle by the cells was evaluated according to the following criteria based on whether or not Fe blue-stained was included in arbitrarily selected 20 cells.
A: Incorporation of Fe (particle) into the interior of a cell membrane could be confirmed in 50% or more (10 or more) cells out of the 20 cells
B: Incorporation of Fe (particle) into the interior of a cell membrane could be confirmed in 10% or more and less than 50% (2 or more and less than 10) cells out of the 20 cells
C: Incorporation of Fe (particle) into the interior of a cell membrane could be confirmed in less than 10% (less than 2) cells out of the 20 cells

### 3-2. Degradability

After 2 days, after 6 days and after 11 days from addition of each particle to a cell culture solution, 0.02 g of a powder obtained by freeze-drying each buffer solution was adopted and a magnetic property measurement system MPMS manufactured by Quantum Design Japan was used to measure saturated magnetic susceptibility (temperature in the system during the measurement: 25°C).

ICP was used to measure the concentration of Fe in each powder.

The saturated magnetic susceptibility was divided by the concentration of Fe, obtained from the same powder, to calculate the saturated magnetic susceptibility per unit amount of the contrast agent.

The average of the saturated magnetic susceptibility per unit amount of the contrast agent, measured with respect to the powder obtained by freeze-drying 6 days later and the saturated magnetic susceptibility per unit amount of the contrast agent, measured with respect to the powder obtained by freeze-drying 11 days later was calculated, and was defined as the long-term residual rate. The saturated magnetic susceptibility per unit amount of the contrast agent measured with respect to the powder obtained by freeze-drying 2 days later was defined as the short-term residual rate. The ratio of the long-term residual rate and the short-term residual rate (long-term residual amount/short-term residual amount) was determined, thereby evaluating degradability of each particle.

### 3-3. Rate of decrease in concentration of particle (day 2 to day 6, day 6 to day 11)

After 2 days, after 6 days and after 11 days from addition of each particle to a cell culture solution, 0.02 g of a powder obtained by freeze-drying each buffer solution was adopted and ICP was used to measure the concentration of Fe in each powder.

The concentration of Fe on day 6 after addition was subtracted from the concentration of Fe on day 2 after addition, and the difference was divided by the number of days (4 days), thereby determining the rate of decrease per day from day 2 to day 6 in the concentration of each particle. The concentration of Fe on day 11 after addition was subtracted from the concentration of Fe on day 6 after addition, and the difference was divided by the number of days (5 days), thereby determining the rate of decrease per day from day 6 to day 11 in the concentration of each particle.

### 3-4. Saturated magnetic susceptibility

After a lapse of 24 hours from addition of each particle to a cell culture solution, 0.02 g of a powder obtained by freeze-drying each buffer solution was adopted and a magnetic property measurement system MPMS manufactured by Quantum Design Japan was used to measure saturated magnetic susceptibility (temperature in the system during the measurement: 25°C).

The results are shown in Table 3 and Table 4.

**[Table 3]**

| | Incorporation of cell | Degradability | Rate of decrease per day in concentration of particle (day 2 to day 6) | Rate of decrease per day in concentration of particle (day 6 to day 11) | Saturated magnetic susceptibility (emu/g) |
|---|---|---|---|---|---|
| Particle 1 | A | 84 | 1.8 | 2.4 | 70 |
| Particle 2 | A | 78 | 0.5 | 3.6 | 75 |
| Particle 3 | A | 73 | 1.3 | 4.0 | 72 |
| Particle 4 | A | 78 | 1.3 | 3.4 | 85 |
| Particle 5 | A | 80 | 0.5 | 3.4 | 80 |
| Particle 6 | A | 71 | 1.3 | 5.0 | 80 |
| Particle 7 | A | 62 | 1.3 | 6.0 | 82 |
| Particle 8 | A | 49 | 1.3 | 8.0 | 85 |
| Particle 9 | A | 64 | 1.3 | 6.0 | 83 |
| Particle 10 | A | 71 | 1.3 | 5.0 | 82 |

**[Table 4]**

| | Incorporation of cells | Degradability | Rate of decrease per day in concentration of particle (day 2 to day 6) | Rate of decrease per day in concentration of particle (day 6 to day 11) | Saturated magnetic susceptibility (emu/g) |
|---|---|---|---|---|---|
| Particle 11 | A | 40 | 2.5 | 10.0 | 90 |
| Particle 12 | A | 46 | 2.5 | 9.0 | 95 |
| Particle 13 | A | 40 | 2.5 | 10.0 | 90 |
| Particle 14 | A | 87 | 1.3 | 2.0 | 72 |
| Particle 15 | A | 51 | 0.5 | 9.0 | 95 |
| Particle 16 | A | 84 | 0.5 | 2.6 | 40 |
| Particle 17 | A | 84 | 0.5 | 2.6 | 43 |
| Particle 18 | A | 88 | 0.5 | 1.8 | 55 |
| Particle 19 | A | 85 | 0.5 | 2.4 | 53 |
| Particle 20 | B | 95 | 0.5 | 0.5 | 55 |
| Particle 21 | B | 98 | 0.5 | 0.0 | 53 |
| Particle 22 | C | (Unmeasurable) | (Unmeasurable) | (Unmeasurable) | 38 |

Particle 1 to particle 19, each including a contrast agent particle and a degradation accelerator contained in the contrast agent particle, was high in biodegradability after a lapse of a certain time from administration. Particle 1 to particle 19 was lower in the rate of decrease per day from day 2 to day 6 in the concentration of each particle than the rate of decrease per day 6 to day 11 in the concentration of each particle, and it was supposed that the speed of dissolution or degradation of each particle, although was low after administration, was increased with time.

In particular, particle 11 to particle 13, where a compound including an easily ionizable atom or a chelating agent and a biodegradable substance were used in combination as a degradation accelerator, was higher in biodegradability after a lapse of a certain time from administration.

Particle 15, where a contrast agent particle containing a degradation accelerator was further covered with a degradation accelerator, was also higher in biodegradability after a lapse of a certain time from administration.

The present application claims the priority based on Japanese Patent Application No. 2017-213709 filed on November 6, 2017, the contents described in the claims, description and accompanying drawings of which are herein incorporated.

### Industrial Applicability

The present invention provides a contrast composite particle high in biodegradability after an image is taken. The composite particle is more rapidly excreted from cells after an image is taken, than a conventional contrast agent, and thus less influences the living body and cells, when used in a contrast application. Cells including the composite particle can be used for transplantation into the living body. An image of the cell can be taken after such transplantation to thereby allow for observation about whether or not the cell (or cell structure) transplanted is colonized in an affected area, without any incision. Thus, the composite particle can also be expected to improve QOL of a transplant patient.

### Reference Signs List

- 100, 200: composite particle
- 110: contrast agent particle
- 112, 112a, 112b: pore
- 120, 130, 220: degradation accelerator
- 210: substance having contrast effect

## Claims

1. A contrast composite particle having a ratio between a long-term residual amount and a short-term residual amount (long-term residual amount/short-term residual amount) of less than 90%, wherein the long-term residual amount is an average of a contrast rate in cells after a lapse of 6 days and a contrast rate in cells of the same species after a lapse of 11 days and the short-term residual amount is a contrast rate in cells of the same species after a lapse of 2 days.

2. The composite particle according to claim 1, comprising:
a substance having a contrast effect; and
a degradation accelerator that is a substance to be more easily dissolved or degraded in cells than the substance having a contrast effect and that is a substance to accelerate dissolution or degradation of the substance having a contrast effect; wherein
the degradation accelerator is encapsulated in the substance having a contrast effect.

3. The composite particle according to claim 2, wherein
the substance having a contrast effect is a substance comprising an atom of a metal element, and
the degradation accelerator comprises a compound comprising an atom of any metal element that is higher in ionization tendency than the metal element, or a chelating agent capable of being coordinately bound to the atom of the metal element.

4. The composite particle according to claim 3, wherein a proportion of a mass of the atom of any metal element that is higher in ionization tendency than the metal element, based on a total mass of components other than water, is 0.1mass% or more and 90mass% or less.

5. The composite particle according to claim 3 or 4, wherein
the metal element is Fe, and
any metal element that is higher in ionization tendency than the metal element is a compound comprising a metal element selected from the group consisting of Mg, Na, Ca, K, Al, and Zn.

6. The composite particle according to claim 2, wherein the degradation accelerator comprises a biodegradable substance.

7. The composite particle according to claim 6, wherein the biodegradable substance is gelatin.

8. The composite particle according to claim 6 or 7, wherein a proportion of a mass of the biodegradable substance based on a total mass of components other than water is 5mass% or more and 95mass% or less.

9. The composite particle according to claim 2, wherein
the substance having a contrast effect is a substance comprising an atom of a metal element, and
the degradation accelerator comprises a compound comprising an atom of any metal element that is higher in ionization tendency than the metal element, or a chelating agent capable of being coordinately bound to the atom of the metal element, and a biodegradable substance.

10. The composite particle according to any one of claims 2 to 9, wherein the substance having a contrast effect is a substance capable of being comprised in a contrast agent for MRI.

11. The composite particle according to claim 10, wherein
the substance having a contrast effect comprises Fe, and
a saturated magnetic amount per gram of Fe at a water content of 0.01mass% or more and 10mass% or less is 1 emu/g or more and 150 emu/g or less.

12. The composite particle according to any one of claims 2 to 11, wherein a volume average particle size of the composite particle is 2.0 nm or more and 1500 nm or less.

13. The composite particle according to any one of claims 2 to 12, wherein an outer edge of the composite particle is covered with the degradation accelerator.

14. A contrast composite particle comprising:
a substance having a contrast effect; and
a degradation accelerator that is a substance to be more easily dissolved or degraded in cells than the substance having a contrast effect and that is a substance to accelerate dissolution or degradation of the substance having a contrast effect; wherein
the degradation accelerator is encapsulated in the substance having a contrast effect.

15. A contrast composite particle in which, in a case where a rate of decrease per unit time in concentration in living cells after administration to the living cell is measured with respect to two periods not overlapping with each other, the rate of decrease at a shorter elapsed time after administration is lower than the rate of decrease at a longer elapsed time after administration.

16. A method for producing the composite particle according to any one of claims 1 to 15, comprising:
preparing a solution comprising a substance having a contrast effect, and a degradation accelerator that is a substance to be more easily dissolved or degraded in cells than the substance having a contrast effect, and
forming a particle of the substance having a contrast effect, in the solution.

17. A cell comprising the composite particle according to any one of claims 1 to 15, in an interior of a cell membrane.

18. A cell structure obtained by collecting a plurality of the cells according to claim 17.

19. The cell structure according to claim 18, wherein the cell structure is a cell sheet where a plurality of the cells are aggregated in the form of a sheet, a spheroid where a plurality of the cells are aggregated in the form of a sphere, a structure where a plurality of the cells are covered with a film comprising a polymer, or a structure where a surface of a steric structure comprising a polymer is covered with a plurality of the cells.

20. A mixed dispersion comprising a plurality of the cells according to claim 17, or the cell structure according to claim 18 or 19, and a polymer.
